# EUROPÄISCHE PATENTSCHRIFT

(11) **EP 0 389 773 B1**
(45) Veröffentlichungstag und Bekanntmachung des Hinweises auf die Patenterteilung: **22.09.1993**
(21) Anmeldenummer: 90102817.5
(22) Anmeldetag: 13.02.1990
(51) Int. Cl.: A61K 47/28, A61K 31/15

(54) **Verfahren zur Herstellung einer Galenischen Zubereitung mit optimaler Bioverfügbarkeit des Wirkstoffs 2-Hydroxy-5-methyllaurophenonoxime (HMLO)**
Process for preparing a galenic formulation of 2-hydroxy-5-methyllaurophenonoxime with optimal bioavailability
Procédé de préparation d'une composition pharmaceutique de phénonoxime-2-hydroxy-5-méthyllauro avec de la biodisponibilité optimale

(30) Priorität: 29.03.1989 DD 326978
(43) Veröffentlichungstag der Anmeldung: 03.10.1990
(73) Patentinhaber: Salutas Fahlberg-List Pharma GmbH, D-39122 Magdeburg (DE)
(72) Erfinder: Lücke, Lothar, Dr., DDR-3090 Magdeburg (DD); Fries, Gerhard, Dr., DDR-3041 Magdeburg (DD); Voigt, Günter, Dr., DDR-3034 Magdeburg (DD); Neubert, Reinhard, Dr., sc., DDR-4090 Halle (DD); Fürst, Walter, Prof.,Dr. sc., DDR-4090 Halle (DD); Slapke, Jürgen, Dr. sc., DDR-1297 Schwanebeck (DD); Schewe, Tankred, Prof., Dr. sc., DDR-1140 Berlin (DD)
(74) Vertreter: Zipse + Habersack

(56) Entgegenhaltungen:
- EP-A- 0 179 583
- EP-A- 0 263 493
- DD-A- 235 450

## Beschreibung

### Anwendungsgebiet der Erfindung

Die Erfindung betrifft ein Verfahren zur Herstellung einer für die orale Applikation geeigneten Arzneiform, die den Wirkstoff HMLO enthält und die Bioverfügbarkeit dieses Wirkstoffs deutlich verbessert und damit die Voraussetzungen zur optimalen Therapie des Asthma bronchiale, von allergischen, rheumatischen und entzündlichen Erkrankungen unterschiedlicher Genese sowie der Thrombose bietet.

### Charakteristik der bekannten technischen Lösungen

Wie bekannt, besteht ein Zusammenhang zwischen der Löslichkeit, der Resorption und der Bioverfügbarkeit einer Substanz. Extrem schwer lösliche Arzneistoffe werden oftmals auch schwer resorbiert. Allgemein ist damit zu rechnen, daß sich bei einer Löslichkeit von weniger als 0,3 % Probleme bezüglich der Bioverfügbarkeit ergeben werden (Ritschel, W.A. Angewandte Biopharmazie, Wissenschaftliche Verlagsgesellschaft MBH Stuttgart 1953, S. 53; Thoma, K. Österreich.-Apotheken-Ztg. 32 (1978) 8, S. 157).
Es ist bekannt, daß die Bioverfügbarkeit schwerlöslicher Arzneistoffe beispielsweise durch chemische Abwandlung des Arzneistoffes, durch Veränderung der Molekülgröße, durch Schaffung geeigneter pH-Verhältnisse, durch geeignete Wahl der Applikationsform, durch Mikronisierung, durch Bereitung sogenannter Feststoffdispersionen sowie durch Verwendung von Resorptionsbeschleunigern beeinflußt werden kann.
So werden nach W.A. Ritschel (Ritschel, W.A. Angewandte Biopharmazie, Wissenschaftliche Verlagsgesellschaft MBH Stuttgart 1973) zur Verbesserung der Bioverfügbarkeit organische Lösungsmittel - wie Ethanol, Fettalkohole und Fettsäureester, oberflächenaktive Stoffe, wie nichtionogene, anionenaktive und kationenaktive Tenside, Saponine, Enzyme, Komplexbildner und Kohlenhydrate, wie Sorbit und Glucosamin, eingesetzt.
Zur Resorptionsbeeinflussung des schwerlöslichen Arzneistoffes HMLO wurden Lösungen und Emulsionen, die zum Beispiel Öle, insbesondere Baumwollsaatöl, Erdnußöl, Cashewnußöl, Maiskeimöl, Olivenöl, Ricinusöl und Sesamöl, enthalten, verwendet (DDR-Patent DD 235 450 A 1). Dadurch kann die Bioverfügbarkeit erhöht werden. Allerdings ist eine solche Zubereitung für die Dauertherapie nicht geeignet, weil dadurch nicht nur der Fettstoffwechsel ungünstig beeinflußt wird, sondern auch durch Auftreten von Diarrhoe die Notwendigkeit zum Abbruch der Therapie gegeben sein kann.
In der gleichen Patentschrift sind eine Vielzahl von weiteren Hilfsstoffen angegeben, die für eine Formulierung verwendet werden können, ohne daß eine Resorptionsbeeinflussung nachgewiesen wird.

### Ziel der Erfindung

Das Ziel der Erfindung besteht darin, die Bioverfügbarkeit des Wirkstoffs HMLO nach oraler Applikation durch den Einsatz von Hilfsstoffen, die die Resorption beeinflussen, zu verbessern.

### Darlegung des Wesens der Erfindung

### - Aufgabenstellung

Der Erfindung lieft die Aufgabe zugrunde, Hilfsstoffe aufzufinden, die die Resorption und damit die Bioverfügbarkeit verbessern ohne die Verträglichkeit der Zubereitung negativ zu beeinflussen.

### - Merkmale der Erfindung

Überraschenderweise wurde gefunden, daß in Gegenwart von Gallensäuren die Resorption des Wirkstoffs HMLO in vitro und in vivo sowohl gegenüber dem reinen Wirkstoff als auch gegenüber allen bekannten galenischen Zubereitungen deutlich verbessert wird. Das gilt auch im Vergleich mit der öligen Zubereitung, obwohl dieser Zubereitung offensichtlich der gleiche Mechanismus der Resorptionsbeeinflussung zugrunde liegt.
Das Öl dient in den öligen Zubereitungen wahrscheinlich nicht als Lösungsvermittler oder Tensid sondern regt die Gallensekretion an und trägt so zur verbesserten Resorption bei. Setzt man die Gallensäure dem Wirkstoff zu, so wird der gleiche bzw. ein quantitativ verbesserter Effekt erzielt, ohne daß dabei die mit der vermehrten Ölzufuhr verbundenen unerwünschten Nebenwirkungen auftreten können.

Als Gallensäuren können beispielsweise Desoxy- oder Dehydrocholsäure oder ein Gemisch beider in Form ihrer Salze im Molverhältnis von Gallensäure zu HMLO wie 0,1 : 1 bis 10 : 1, bevorzugt 0,5 : 1 bis 1 : 0,5, verwendet werden.

Die Formulierung des Wirkstoffs HMLO mit der Gallensäure bzw. dem Gemisch der Gallensäuren kann in Form von Lösungen, Suspensionen, Kapseln, Granulaten, Tabletten oder Dragees, bevorzugt in Form von Granulaten oder Tabletten, erfolgen.

Zweckmäßigerweise wird dazu der HMLO-Wirkstoff mit dem Salz der Gallensäure bzw. -säuren und den üblichen Tablettierhilfsstoffen, wie beispielsweise Milchzucker, Kartoffelstärke und Zucker, homogen vermischt und anschließend mit einer Polyvinylalkohollösung granuliert. Das erhaltene Granulat wird getrocknet, gesiebt und mit entsprechenden Gleit- und Fließregulierungsmitteln, wie beispielsweise Calciumstearat und Talk, vermischt. Das erhaltene Granulat kann entweder direkt appliziert oder zu Tabletten verpreßt werden.

Die nachfolgenden Beispeile erläutern die Erfindung, ohne sie jedoch dadurch einzuschränken.

### Ausführungsbeispiele

### Beispiel 1

| Zusammensetzung der Rezeptur | |
|---|---|
| 360 g | HMLO-Wirkstoff |
| 240 g | Natriumdesoxycholat |
| 214 g | Milchzucker |
| 100 g | Kartoffelstärke |
| 10 g | Zucker |
| 9 g | Gelatine |
| 30 g | Calciumstearat |
| 37 g | Talk |

360 g HMLO-Wirkstoff, 240 g Natriumdesoxycholat, 214 g Milchzucker, 100 g Kartoffelstärke und 10 g Zucker werden trocken, z.B. im Wirbelschichtgranulator, vermischt und durch Einsprühen von 250 ml einer wäßrigen Gelatinelösung (9 g Gelatine), die eine Temperatur von ca. 333 K aufweist, granuliert. Das so erhaltene Granulat wird gesiebt und mit der äußeren Phase, bestehend aus 30 g Calciumstearat und 37 g Talk, homogen vermischt. Das erhaltene Produkt kann direkt als Applikationsform verwendet oder zu Tabletten mit einem Gewicht von 200 mg und einen Wirkstoffgehalt von 100 mg verpreßt werden.

### Beispiel 2

| Zusammensetzung der Rezeptur | |
|---|---|
| 500 g | HMLO-Wirkstoff |
| 120 g | Natriumdehydrocholat |
| 204 g | Milchzucker |
| 90 g | Kartoffelstärke |
| 20 g | Zucker |
| 9 g | Gelatine |
| 20 g | Calciumstearat |
| 37 g | Talk |

500 g HMLO-Wirkstoff, 120 g Natriumdehydrocholat, 204 g Milchzucker, 90 g Kartoffelstärke und 20 g Zucker werden trocken, z.B. im Wirbelschichtgranulator, vermischt und durch Einsprühen von 250 ml einer wäßrigen Galatinelösung (9 g Gelatine), die eine Temperatur von ca. 333 K aufweist, granuliert. Das erhaltene Granulat wird gesiebt und mit der äußeren Phase, bestehend aus 20 g Calciumstearat und 37 g Talk, homogen vermischt. Das so erhaltene Produkt kann direkt als Applikationsform verwendet oder zu Tabletten mit einem Gewicht von 400 mg und einem Wirkstoffgehalt von 200 mg verpreßt werden.

### Beispiel 3

| Einfluß verschiedener Hilfsstoffe auf die Löslichkeit von HMLO-Wirkstoff in Wasser bei 293 K | | |
|---|---|---|
| Hilfsstoff | Hilfsstoffkonzentration in % | Löslichkeit (µg . ml⁻¹) |
| Na-Desoxycholat | 0,75 | 2750,0 |
| Tween^{®} 80 | 0,75 | 130,0 |
| Benzalkoniumbromid | 1,00 | 24,0 |
| PVA | 10,00 | - |
| Propylenglycol | 1,00 | 2,3 |
| Polyethylenglycol 6500 | 10,00 | 1,0 |
| Heweten 40 | 10,00 | - |
| Reiner HMLO-Wirkstoff ist in Wasser nicht löslich. | | |

### Beispiel 4

| Beeinflussung der Resorption von HMLO-Wirkstoff in Abhängigkeit von verwendeten Hilfsstoffen in vivo am Kaninchen nach oraler Applikation | | | | |
|---|---|---|---|---|
| Zubereitung | Dosis (mg.kg⁻¹) | Anzahl d. Tiere | MRT (h)^{24 h} | AUC (µg.h.ml⁻¹) |
| HMLO-Wirkstoff ohne Hilfsstoffe | 12 | 4 | - | - |
| ölige Zubereitung | 12 | 6 | 14,7 | 18,7 |
| Desoxycholsäurezubereitung | 12 | 6 | 13,6 | 19,4 |

Es zeigt sich, daß HMLO-Wirkstoff ohne Hilfsstoffe nicht in einem meßbaren Ausmaß resorbiert wird. Durch Verwendung von Öl als Hilfsstoff ist eine deutliche Resorptionsbeeinflussung zu erreichen, die allerdings durch die Verwendung von Gallensäuren noch deutlich übertroffen wird.

## Patentansprüche

1. Verfahren zur Herstellung einer galenischen Zubereiung mit optimaler Bioverfügbarkeit des Wirkstoffes 2-Hydroxy-5-methyllaurophenonoxim (HMLO), dadurch gekennzeichnet, daß Gallensäuren als Resorptionsmittel verwendet werden.

2. Verfahren nach Anspruch 1, dadurch gekennzeichnet, daß als Gallensäure Desoxycholsäure oder Dehydrocholsäure entweder einzeln oder im Gemisch verwendet werden.

3. Verfahren nach Anspruch 1 und 2, dadurch gekennzeichnet, daß die Gallensäuren im Molverhältnis von Gallensäure zu HMLO-Wirkstoff wie 0,1 : 1 bis 10 : 1, bevorzugt 0,5 : 1 bis 1 : 0,5 verwendet werden.

## Claims

1. Method of producing a galenical preparation with optimum bioavailability of the active substance 2-hydroxy-5-methyllaurophenonoxime (HMLO), characterised in that bile acids are used as resorption agents.

2. Method as claimed in claim 1, characterised in that deoxycholic acid or dehydrocholic acid are used either alone or in a mixture as bile acids.

3. Method as claimed in claim 1 or 2, characterise din that the bile acids are used in a molar ratio of bile acid of HMLO active substance of 0.1 : 1 to 10 : 1, preferably 0.5 : 1 to 1 : 0.5.

## Revendications

1. Procédé de préparation d'une composition galénique à biodisponibilité optimale du principe actif 2-hydroxy-5-méthyllaurophénonoxime (HMLO), caractérisé en ce qu'on utilise des acides biliaires comme agent de résorption.

2. Procédé selon la revendication 1, caractérisé en ce qu'on utilise comme acide biliaire de l'acide désoxycholique ou de l'acide déshydrocholique soit seuls soit en mélange.

3. Procédé selon la revendication 1 et la revendication 2, caractérisé en ce qu'on utilise les acides biliaires en proportion molaire de l'acide biliaire au principe actif HMLO de 0,1:1 à 10:1, de préférence 0,5:1 à 1:0,5.
